# EUROPEAN PATENT SPECIFICATION

(11) **EP 1 337 224 B1**
(45) Date of publication and mention of the grant of the patent: **18.10.2006**
(21) Application number: 01982513.2
(22) Date of filing: 09.11.2001
(51) Int. Cl.: A61Q 19/00, A61Q 19/08, A61K 8/92

(54) **COSMETIC COMPOSITIONS CONTAINING CLOUDBERRY (RUBUS CHAMAEMORUS) SEED OIL OBTAINED BY SUPERCRITICAL CARBON DIOXIDE EXTRACTION**
KOSMETISCHE ZUSAMMENSETZUNGEN ENTHALTEND MIT SUPERKRITISCHEM KOHLENDIOXID EXTRAHIERTES TORFBEERENSAMENÖL (RUBUS CHAMAEMORUS)
COMPOSITIONS COSMETIQUES A BASE D'HUILE DE RONCE PETIT-MURIER (RUBUS CHAMAEMORUS) OBTENUE PAR EXTRACTION AVEC DU DIOXIDE DE CARBONE EN PHASE SUPERCRITIQUE

(30) Priority: 10.11.2000 FI 20002470
(43) Date of publication of application: 27.08.2003
(73) Proprietor: Lumene Oy, 02780 Espoo (FI)
(72) Inventor: ISOHANNI, Tiina, FIN-02780 ESPOO (FI); KOLUNEN, Leena, FIN-00200 Helsinki (FI)
(74) Representative: Grew, Eva Regina
(86) International application number: PCT/FI2001/000975
(87) International publication number: WO 2002/038109

(56) References cited:
- WO-A1-91/18588
- WO-A1-98/13021
- FR-A1- 2 720 901
- FR-A1- 2 795 958
- DATABASE PROMT [Online] KLEPACKI LAURA: 'Tova's calm approach to beaty', XP002908042 Retrieved from STN Database accession no. 1999:692716 & WWD 15 October 1999, page 10
- DATABASE WPI Week 199948, Derwent Publications Ltd., London, GB; AN 1991-284723, XP002908043 & JP 2 962 558 B2 (SHISEIDO CO LTD) 12 October 1999
- DATABASE WPI Week 200035, Derwent Publications Ltd., London, GB; AN 2000-402798, XP002908044 & JP 2000 007550 A (SHISEIDO CO LTD) 11 January 2000
- PEKKA MANNINEN ET AL.: 'Large-scale supercritical carbon dioxide extraction and supercritical carbon dioxide countercurrent extraction of cloudberry seed oil' J. AGRIC. FOOD. CHEM. vol. 45, no. 7, 1997, pages 2533 - 2538, XP000656901
- P. MANNINEN ET AL.: 'Supercritical fluid chromatography-gas chromatography of volatiles in cloudberry (Rubus chamaemorus) oil extracted with supercritical carbon dioxide' JOURNAL OF CHROMATOGRAPHY A vol. 787, no. 1-2, 1997, pages 276 - 282, XP004097612
- DATABASE WPI Week 200120, Derwent Publications Ltd., London, GB; AN 2001-194007, XP002908045 & JP 2000 327555 A (SHISEIDO CO LTD) 28 November 2000

## Description

### FIELD OF THE INVENTION

The present invention is directed to cosmetic compositions containing cloudberry (Rubus chamaemorus) seed oil. Cloudberry seed oil can be used, for example, in emulsion creams, lipsticks, hair cosmetics products, as well as in products for skin hygiene.

### BACKGROUND OF THE INVENTION

Most plant oils contain unsaturated fatty acids, such as oleic, linoleic and linolenic acids to various degrees. Also the seeds of berries are known to contain oils, the major component of which are the triacylglycerols, which form an important energy reserve for germinating seeds. The fatty acid composition of seed oil triacylglycerols varies widely among plant species, and tends to be characteristic of particular plant families.

The fatty acid composition of the seed oils of edible berries has been studied. One study included the analysis of 22 common edible berries including cloudberry (Johansson A. et al., Z. Lebensm. Unters. Forsch. A (1997) 204; 300-307). In this study the seeds were separated from the berries, dried and crushed, and the oil extracted with a mixture of chloroform and methanol, and the eluted neutral lipids analysed for their fatty acid composition. Solvent extraction methods are, however, not applicable for the preparation of substances intended for cosmetic use, due to an insufficient degree of purity and the inclusion of solvent residues. Manninen et al (Manninen, P. et al., J. Agric. Food Chem. 1997, 45, 2533-2538) describe the use of supercritical fluid extraction for the extraction of oil from cloudberry seeds in order to determine its composition.

The present invention is directed to cosmetic, compositions which contain cosmetically acceptable substances and, in addition, cloudberry seed oil prepared by supercritical fluid extraction.

According to the invention it has now been found that cloudberry seed oil which is prepared by supercritical fluid, especially carbon dioxide extraction is especially advantageous for inclusion in cosmetic compositions. In this way, many of the valuable components in cloudberry seed oil can be taken advantage of as cloudberry seed oil extracted in this way retains the natural volatile components, including the components responsible for the odor and the aromatic components with a preservative effect. In addition, it is possible to utilize the natural colour of the oil so extracted. In the cosmetic compositions according to the invention it is thus possible to reduce or even eliminate the need to add additional ingredients. The oil obtained by supercritical fluid extraction rather than by solvent extraction or distillation, is to its composition, appearance and aroma closer to the natural fractions than cloudberry seed oil prepared in accordance with other methods, such as solvent extraction. In addition it will be sufficiently pure for cosmetic purposes.

### DETAILED DESCRIPTION OF THE INVENTION

According to the invention, the cloudberry seed oil included in the cosmetic compositions is prepared by supercritical fluid, typically carbon dioxide extraction. As stated above, supercritical fluid extraction of berries is known as such, as are also the process conditions used. Supercritical fluid extraction can be carried out in a range of temperatures and pressures, but typically a fairly moderate temperature is used, such as a temperature from around room temperature to 40-50 °C, and a pressure up to 30 MPa. This is well known to a person skilled in the art. By varying the process conditions it is possible to some extent to vary the composition, such as the lipid composition of the extracted oil. According to the invention it is, however, possible to secure that the product obtained contains, in addition to the desired fatty acids, the volatile compounds which are responsible for the odor of the oil and the preservative effect, as well as those ingredients which give the desired colour. By using supercritical carbon dioxide extinction it is thus possible to retain in the product the volatile compounds, which amount to appr. 4 to 5 % of the oil.

In the composition according to the invention it is advantageous to use oil which contains all possible fractions together. Typical components in cloudberry seed oil are linoleic and alpha-linolenic acids, carotenoids and plant sterols, as well as aromatic volatile compounds. Components which are advantageous for the composition according to the invention are, for example, carotenoids having anti-oxidant activity, and, in addition, providing the desired colour, free fatty acids, and volatile aromatic compounds, such as cinnamyl derivatives. These cinnamyl compounds give the cloudberry seed oil its typical odor. Only a few natural oils are equally fragrant as cloudberry seed oil. Depending on the concentration used, the cloudberry seed oil fragrance is retained by and pleasantly apparent also in the cosmetic composition according to the invention. In addition, cloudberry seed oil contains small amounts of benzoic acid, which is a typical preservative in cosmetics. Cloudberry seed oil is golden yellow or slightly orange to its colour and it also gives a pleasing natural yellow colour to the emulsion prepared. Thus many of the advantageous and desired properties of the cosmetic compositions according to the invention are derived from the extracted cloudberry seed oil.

Cloudberry seed oil and the composition containing the same have many advantageous properties and effects, such as skin and hair protecting and moisturizing effect, elasticity maintaining and/or enhancing effect as well as skin softening effect. In addition, cloudberry oil as well as the cosmetic compositions containing the same seem to possess both antioxidant activity as well as so called anti-wrinkle effect.

The cosmetic composition according to the invention contains, depending on the intended use of the composition, cloudberry seed oil preferably 0.01 to 20 % by weight.

The cosmetic composition according to the invention can be, for example, an emulsion cream, a lipstick, a hair cosmetics product or a product to be used for skin hygiene.

Such compositions can contain cosmetically acceptable substances or agents which are per se known to and chosen by the person skilled in the art according to the specific cosmetic composition desired. An illustrative list of such acceptable conventional substances are described in the following and in the examples.

The emulsion cream according to the invention containing cloudberry seed oil can be of the type oil-in-water emulsion, water-in-oil emulsion, water-oil-water emulsion or a microemulsion. The emulsion cream composition according to the invention contains cloudberry seed oil preferably 0.01 to 20 % by weight, more preferably 0.01 to 10 % by weight and most preferably 1 to 5 % by weight.

In the emulsions according to the invention the cloudberry seed oil can be combined with betaine. In addition to moisturizing, betaine acts as a stabilizing factor in water-oil-water emulsions and regulates the viscosity in oil-in-water emulsions. In addition, if the emulsion also contains pigments or solid particles, betaine acts as a dispersing factor for these. Betaine is included in the emulsion preferably in an amount of 1 to 10 % by weight, more preferably 2 to 5 % by weight. In the description of the invention as well as in the claims, betaine is intended to mean a compound with the chemical name trimethylglycine or trimethylammonium acetate.

In the emulsion creams according to the invention the cloudberry seed oil can be combined also with either synthetic or natural vitamins. The cloudberry seed oil can be combined with retinol and A-vitamin palmitate. On the other hand, the cloudberry seed oil can be combined with different E-vitamins and E-vitamin derivatives, C-vitamin and its derivatives, panthenol and other B-vitamins and/or biotine. The composition contains synthetic or natural vitamins preferably 0.01 to 10 % by weight, more preferably 0.02 to 5 % by weight.

The emulsion cream composition according to the invention contains, in addition, one or more adjuvants acceptable in the field of cosmetics, such as preservation agent, thickening agent and other suitable additives, such as for example perfumes and/or colouring agents. Suitable preservation agents are for example parabens, phenoxyethanol, imidazolidinyl urea and methyldibromo glutaronitrile. These preservation agents can be used alone or combined with each other. As thickening agent any thickening agent suitable in the field of cosmetics can be used, as long as it is compatible with the other components of the composition, for example xantane gum and hydroxyethylcellulose, hydroxypropylmethylcellulose, Scelorotium gum, Chondrus Crispus, polyacrylates, polyacrylamides and magnesium aluminium silicate. Thickening agents can be used alone or in combination with each other.

In addition, one or more compounds acting as an emulsifier, i.e. a compound dispersing and stabilizing the oil in water, is needed in the emulsion cream composition. Useful emulsifiers are all non-ionic emulsifiers accepted by the cosmetic legislation, such as, for example, glyceryl stearate, PEG-5 glyceryl stearate, PEG-100 stearate, PEG-30 dipolyhydroxystearate, hydrogenated lecithine and PEG-8 bees wax, steareth-21, steareth-2, sorbitan olivate as well as a mixture of a fatty glucoside, such as for example cetearyl, cocoyl, or myristyl glucoside and a fatty alcohol, such as for example cetearyl, cetyl, stearyl or myristyl alcohol. In addition, of anionic emulsifieras, for example stearic acid, sodium hydroxide and triethanolamine are useful.

According to the invention cloudberry seed oil can be used also in the preparation of lipstick compositions. The lipstick compositions in accordance with the invention contain cloudberry seed oil 0.01 to 20 % by weight, preferably 0.5 to 10 % by weight and most preferably 1 to 5 % by weight.

The lipstick composition according to the invention contains in addition to cloudberry seed oil waxes, oils, colouring and pearlescent agents which are acceptable in the field of cosmetics and which are traditional components of lipstick compositions. Usable waxes are the natural waxes, such as bees wax, candelilla, carnauba, cereal based waxes, jojoba wax and their derivatives, in addition paraffin waxes and synthetic polyethylenes. Another lipstick composition according to the invention is an emulsion composition which contains in addition to cloudberry seed oil water, betaine and/or xylitol as a moisturizing agent and one or more emulsifiers acceptable in the field of cosmetics. Betaine is included in the composition preferably in an amount of 0.1 to 5 % by weight, more preferably 1 to 3 % by weight. Xylitol is on the other hand included in the composition preferably 0.1 to 5 % by weight, more preferably 1 to 3 % by weight.

The lipstick compositions according to the invention can in addition contain one or more adjuvants and/or additives acceptable in the field of cosmetics, such as preservation agent. Suitable preservation agents are for example parabens, phenoxyethanol. These preservation agents can be used alone or in combination with each other.

Cloudberry seed oil can be used also in the preparation of hair cosmetics products. It can be used in wash and care products to impart shine to the hair and to improve the manageability of the hair. In addition, it can be used in products intended for special treatment of the scalp to treat and to moisturize the scalp. In addition cloudberry seed oil can be used for products intended for skin hygiene in order to impart elasticity and moisture to the skin.

The compositions according to the invention intended for washing the hair or the skin or for hair or scalp care contain cloudberry seed oil preferably in an amount of 0.01 to 20 % by weight, more preferably 0.1 to 20 % by weight, and most preferably 1 to 5 % by weight.

The compositions according to the invention intended for washing the hair or the skin contain, in addition to the washing substances, detergents, such as for example sodium lauryl ether sulphate, decyl glucoside, cococamidopropyl betaine, one or more adjuvants acceptable in the cosmetic field, such as a preservation agent, thickener and other suitable additives, such as perfumes and/or colouring agents. Suitable preservation agents are for example the parabens, phenoxyethanol, 5-bromo-5-nitro-1,3-dioxane and methyldibromo glutaronitrile. These preservation agents can be used alone or in combination with each other. As thickeners, any thickener suitable in the field of cosmetics can be used, as long as it is compatible with the other components of the composition, as examples sodium chloride and PEG-7 glyceryl cocoate can be mentioned.

The compositions according to the invention intended for hair or scalp care contain a cationically active substance, such as for example cetrimonium chloride, in addition an emulsion forming substance, such as for example cetyl alcohol, cetearyl alcohol, ceteareth-20. In addition, the compositions intended for hair or scalp care can contain one or more adjuvants acceptable in the field of cosmetics, such as a cellulose derivative, ethanol and/or water.

The invention is illustrated by means of the following examples.

### Example 1

A ready-for-use emulsion cream composition for facial and throat skin care contains

| | % by weight |
|---|---|
| Aqua | add 100 |
| cyclopentasiloxane | 6.2 |
| steareth-2 | 4.0 |
| betaine | 3.4 |
| steareth-21 | 2.0 |
| octyldodecanol | 2.0 |
| stearic acid | 2.0 |
| cetearyl alcohol | 1.5 |
| caprylic/capric triglyceride | 1.0 |
| peg-30 dipolyhydroxystearate | 1.0 |
| tocopheryl acetate | 1.0 |
| Rubus chamaemorus, seed oil | 0.01-10 |
| phenoxyethanol | 0.74 |
| ppg-15 stearyl ether | 0.60 |
| panthenol | 0.50 |
| perfume | 0.35 |
| dimethicone/ vinyldimethicon crosspolymer | 0.28 |
| phospholipids | 0.20 |
| chondrus crispus | 0.16 |
| parabens | 0:28 |
| magnesium ascorbyl phosphate | 0.10 |
| peg-8 | 0.06 |
| tocopherol | 0.03 |
| glycerin | 0.025 |
| ascorbyl palmitate | 0.005 |
| sodium chloride | 0.003 |
| ascorbic acid | 0.001 |

The described emulsion cream composition moisturizes, brightens and softens the skin. In addition it has anti-oxidant properties.

### Example 2

A ready-for-use emulsion cream composition for facial and throat skin care:

| | |
|---|---|
| Aqua | add 100 |
| sorbitan olivate | 15.0 |
| vegetable oil | 7.00 |
| squalane | 11.0 |
| cyclopentasiloxane | 7.6 |
| decyl cocoate | 3.0 |
| glycerin | 2.20 |
| tocopheryl acetate | 2.0 |
| sodium chloride | 1.5 |
| c20-40 alcohols | 1.0 |
| Rubus chamaemorus, seed oil | 0.01-10 |
| c20-40 acid | 0.85 |
| phenoxyethanol | 0.72 |
| aluminium/magnesium hydroxide stearate | 0.69 |
| dimethicone/vinyldimethicone | |
| crosspolymer | 0.28 |
| perfume | 0.20 |
| parabens | 0.28 |
| polyethylene | 0.15 |
| phospholipids | 0.15 |
| retinol | 0.03 |
| tocopherol | 0.03 |
| ascorbyl palmitate | 0.005 |
| ascorbic acid | 0.001 |
| citric acid | 0.001 |
| retinyl palmitate | 0.00001 |

The emulsion cream composition improves the revitalization of the skin. In addition, it reduces, softens and smoothes fine lines in the skin.

### Example 3

A lipstick composition (emulsion) according to the invention

| | |
|---|---|
| | % |
| Ricinus communis | add 100 |
| squalane | 17.5 |
| candelilla cera | 12.1 |
| apricot kernel oil peg-6 esters | 8.0 |
| acetylated lanolin | 5.9 |
| Aqua | 5.0 |
| peg-8 beeswax | 4.7 |
| hydrogenated polydecene | 4.0 |
| butyrospermum parkii | 4.0 |
| isopropyl myristate | 3.9 |
| cera alba | 2.3 |
| ethylcellulose | 2.0 |
| xylitol/betaine | 2.0 |
| polyethylene | 2.0 |
| mica | 1.8 |
| arachidyl alcohol | 1.4 |
| behenyl alcohol | 1.3 |
| arachidyl glucoside | 1.3 |
| perfume | 1.0 |
| Rubus chamaemorus, seed oil | 1.0 |
| carnauba | 0.9 |
| propylparaben | 0.1 |
| peg-8 | 0.1 |
| tocopherol | 0.035 |
| ascorbyl palmitate | 0.006 |
| ascorbic acid | 0.001 |
| citric acid | 0.001 |
| colours | 4.8 |

The lipstick composition of emulsion type according to the invention moisturizes the lips and provides lip care. The end result on the lips is semi-matte.

### Example 4

A lipstick composition according to the invention

| | % |
|---|---|
| Ricinus communis | add 100 |
| oleyl alcohol | 20.0 |
| candelilla cera | 9.8 |
| cera alba | 5.7 |
| acetylated lanolin | 4.7 |
| isopropyl lanolate | 4.0 |
| isopropyl myristate | 3.2 |
| hydrogenated polydecene | 3.0 |
| ethylhexyl methoxycinnamate | 2.0 |
| mica | 1.8 |
| microcrystalline wax | 1.6 |
| Rubus chamaemorus, seed oil | 1.0 |
| perfume | 1.0 |
| carnauba | 0.7 |
| propylparaben | 0.09 |
| peg-8 | 0.04 |
| tocopherol | 0.02 |
| ascorbyl palmitate | 0.003 |
| ascorbic acid | 0.001 |
| citric acid | 0.001 |
| colours | 4.8 |

The lipstick composition according to the invention has a lip protecting and caring effect. It imparts to a high gloss to the lips.

### Example 5

Use of cloudberry seed oil in hair cosmetics

### a. washing products

| Basic formula | % by weight |
|---|---|
| water | ad 100 |
| detergents, e.g. NaLES | 5-30 |
| co-tensides, e.g. cocamidopropyl betaine | 1-10 |
| thickeners, e.g. cellulose derivatives | 0.1-10 |
| moisturizers, e.g. sorbitol | 0.1-10 |
| caring agents, e.g. cloudberry seed oil preservatives, pH regulating agent, colour, perfume | 0.1-20 |

Cloudberry seed oil can be used in washing products to impart gloss to the hair and to improve the manageability of the hair.

### b. caring or conditioning products

| basic formula | % by weight |
|---|---|
| water | ad 100 |
| emulsifier, e.g. ceteareth 20 | 0.1-10 |
| oils, waxes and fatty alcohols | 0.1-10 |
| thickeners, e.g. cellulose derivatives | 0.1-10 |
| cationic surfactants, e.g. cetrimonium chloride | 0.1-15 |
| caring oils (e.g. cloudberry seed oil) | 0.1-20 |
| moisturizers, e.g. sorbitol | 0.1-10 |
| polymers | 0.1-10 |
| preservation agents, pH regulating agents, perfume, colour | |

Cloudberry seed oil can be used in caring products to impart gloss to the hair and to improve the manageability of the hair.

### c. special caring products for scalp treatment

| basic formula | % by weight |
|---|---|
| water | ad 100 |
| ethanol | 10-60 |
| moisturizing agents, e.g. sorbitol | 0.1-10 |
| caring agents, e.g cloudberry seed oil preservation agent, pH regulating agents, colour, perfume | 0.1-20 |

Products intended for special care are emulsions or creams which impart a caring and moisturizing effect to the scalp by means of the cloudberry seed oil.

### Example 6

### Products for washing the skin

| Basic formula | % by weight |
|---|---|
| water | ad 100 |
| detergents, e.g. Na LES | 5-30 |
| co-tensides, e.g. cocamidopropyl betaine | 1-10 |
| thickeners, e.g. cellulose derivatives | 0.1-10 |
| moisturizers, e.g. sorbitol | 0.1-10 |
| caring agents, e.g. cloudberry seed oil preservation agent, pH regulating agent, colour, perfume | 0.1-20 |

Cloudberry seed oil can be used in washing products to impart elasticity and moisture to the skin.

### Example 7

### Deodorizing and antiperspiration products

| Basic formula | % by weight |
|---|---|
| water | ad 100 |
| emulsifiers, e.g. ceteareth-20 | 0.1-10 |
| thickeners, e.g. cellulose derivatives | 0.1-10 |
| ethanol | 0.1-50 |
| cloudberry seed oil | 0.1-15 |
| antimicrobial agent, e.g. farnesol | 0.1-10 |
| antiperspiration agent | 0.1-30 |
| pH regulating agent, colour, perfume | |

## Claims

1. Cosmetic composition which contains cosmetically acceptable substances, **characterized in that** it in addition contains cloudberry seed oil prepared by supercritical fluid extraction.

2. The cosmetic composition according to claim 1, **characterized in that** the supercritical fluid extraction is supercritical carbon dioxide extraction.

3. The composition according to claim 1 or 2, **characterized in that** it contains 0.01 to 20 % by weight of cloudberry seed oil.

4. The composition according to any one of claims 1 to 3, **characterized in that** it is an emulsion cream composition.

5. The composition according to claim 4, **characterized in that** it contains 0.01 to 10 % by weight of cloudberry seed oil.

6. The composition according to claim 5, **characterized in that** it contains 1 to 5 % by weight of cloudberry seed oil.

7. The composition according to any one of claims 4 to 6, **characterized in that** it in addition contains betaine.

8. The composition according to any one of the claims 4 to 7, **characterized in that** in addition contains one or more synthetic or natural vitamins.

9. The composition according to any one of the claims 1 to 3, **characterized in that** it is a lipstick composition.

10. The composition according to claim 9, **characterized in that** it contains 0.5 to 20 % by weight of cloudberry seed oil.

11. The composition according to claim 10, **characterized in that** it contains 1 to 5 % by weight of cloudberry seed oil.

12. The composition according to any one of claims 9 to 11, **characterized in that** it in addition contains betaine.

13. The composition according to any one of claims 9 to 12, **characterized in that** it in addition contains xylitol.

14. The composition according to any one of claims 1 to 3, **characterized in that** it is a composition intended for washing hair or for hair and/or scalp care.

15. The composition according to claim 14, **characterized in that** it contains 0.1 to 20 % by weight of cloudberry seed oil.

16. The composition according to claim 15, **characterized in that** it contains 1 to 5 % by weight of cloudberry seed oil.

17. The composition according to any one of the claims 1 to 3, **characterized in that** it is a composition intended for skin hygiene.

18. The composition according to claim 17, **characterized in that** it contains 0.1 to 20 % by weight of cloudberry seed oil.

19. The composition according to claim 18, **characterized in that** it contains 1 to 5 % by weight of cloudberry seed oil.

## Patentansprüche

1. Kosmetische Zusammensetzung, die kosmetisch annehmbare Substanzen enthält, **dadurch gekennzeichnet, dass** sie zusätzlich durch superkritische Fluidextraktion hergestelltes Moltebeerensamenöl enthält.

2. Kosmetische Zusammensetzung gemäß Anspruch 1, **dadurch gekennzeichnet, dass** die superkritische Fluidextraktion eine superkritische Kohlenstoffdioxidextraktion ist.

3. Zusammensetzung gemäß Anspruch 1 oder 2, **dadurch gekennzeichnet, dass** sie 0,01 bis 20 Gew.-% Moltebeerensamenöl enthält.

4. Zusammensetzung gemäß einem der Ansprüche 1 bis 3, **dadurch gekennzeichnet, dass** sie eine Emulsionscremezusammensetzung ist.

5. Zusammensetzung gemäß Anspruch 4, **dadurch gekennzeichnet, dass** sie 0,01 bis 10 Gew.-% Moltebeerensamenöl enthält.

6. Zusammensetzung gemäß Anspruch 5, **dadurch gekennzeichnet, dass** sie 1 bis 5 Gew.-% Moltebeerensamenöl enthält.

7. Zusammensetzung gemäß einem der Ansprüche 4 bis 6, **dadurch gekennzeichnet, dass** sie zusätzlich Betain enthält.

8. Zusammensetzung gemäß einem der Ansprüche 4 bis 7, **dadurch gekennzeichnet, dass** sie zusätzlich ein oder mehrere synthetische oder natürliche Vitamine enthält.

9. Zusammensetzung gemäß einem der Ansprüche 1 bis 3, **dadurch gekennzeichnet, dass** sie eine Lippenstiftzusammensetzung ist.

10. Zusammensetzung gemäß Anspruch 9, **dadurch gekennzeichnet, dass** sie 0,5 bis 20 Gew.-% Moltebeerensamenöl enthält.

11. Zusammensetzung gemäß Anspruch 10, **dadurch gekennzeichnet, dass** sie 1 bis 5 Gew.-% Moltebeerensamenöl enthält.

12. Zusammensetzung gemäß einem der Ansprüche 9 bis 11, **dadurch gekennzeichnet, dass** sie zusätzlich Betain enthält.

13. Zusammensetzung gemäß einem der Ansprüche 9 bis 12, **dadurch gekennzeichnet, dass** sie zusätzlich Xylitol enthält.

14. Zusammensetzung gemäß einem der Ansprüche 1 bis 3, **dadurch gekennzeichnet, dass** sie eine für das Haarwaschen oder für die Haar- und/oder Kopfhautpflege bestimmte Zusammensetzung ist.

15. Zusammensetzung gemäß Anspruch 14, **dadurch gekennzeichnet, dass** sie 0,1 bis 20 Gew.-% Moltebeerensamenöl enthält.

16. Zusammensetzung gemäß Anspruch 15, **dadurch gekennzeichnet, dass** sie 1 bis 5 Gew.-% Moltebeerensamenöl enthält.

17. Zusammensetzung gemäß einem der Ansprüche 1 bis 3, **dadurch gekennzeichnet, dass** sie eine für die Hautpflege bestimmte Zusammensetzung ist.

18. Zusammensetzung gemäß Anspruch 17, **dadurch gekennzeichnet, dass** sie 0,1 bis 20 Gew-% Moltebeerensamenöl enthält.

19. Zusammensetzung gemäß Anspruch 18, **dadurch gekennzeichnet, dass** sie 1 bis 5 Gew.-% Moltebeerensamenöl enthält.

## Revendications

1. Composition cosmétique qui contient des substances acceptables sur le plan cosmétique, **caractérisée en ce qu'**elle contient en outre de l'huile de graines de ronce petit mûrier préparée par extraction de fluide supercritique.

2. Composition cosmétique selon la revendication 1, **caractérisée en ce que** l'extraction de fluide supercritique est l'extraction au dioxyde de carbone supercritique.

3. Composition selon la revendication 1 ou 2, **caractérisée en ce qu'**elle contient 0,01 à 20 % en poids d'huile de graines de ronce petit mûrier.

4. Composition selon l'une quelconque des revendications 1 à 3, **caractérisée en ce qu'**elle constitue une composition de crème en émulsion.

5. Composition selon la revendication 4, **caractérisée en ce qu'**elle contient 0,01 à 10 % en poids d'huile de graines de ronce petit mûrier.

6. Composition selon la revendication 5, **caractérisée en ce qu'**elle contient 1 à 5 % en poids d'huile de graines de ronce petit mûrier.

7. Composition selon l'une quelconque des revendications 4 à 6, **caractérisée en ce qu'**elle contient en outre de la bétaïne.

8. Composition selon l'une quelconque des revendications 4 à 7, **caractérisée en ce qu'**elle contient en outre une ou plusieurs vitamines synthétiques ou naturelles.

9. Composition selon l'une quelconque des revendications 1 à 3, **caractérisée en ce qu'**elle constitue une composition pour rouge à lèvres.

10. Composition selon la revendication 9, **caractérisée en ce qu'**elle contient 0,5 à 20 % en poids d'huile de graines de ronce petit mûrier.

11. Composition selon la revendication 10, **caractérisée en ce qu'**elle contient 1 à 5 % en poids d'huile de graines de ronce petit mûrier.

12. Composition selon l'une quelconque des revendications 9 à 11, **caractérisée en ce qu'**elle contient en outre de la bétaïne.

13. Composition selon l'une quelconque des revendications 9 à 12, **caractérisée en ce qu'**elle contient en outre du xylitol.

14. Composition selon l'une quelconque des revendications 1 à 3, **caractérisée en ce qu'**elle constitue une composition prévue pour laver les cheveux ou pour le soin des cheveux et/ou du cuir chevelu.

15. Composition selon la revendication 14, **caractérisée en ce qu'**elle contient 0,1 à 20 % en poids d'huile de graines de ronce petit mûrier.

16. Composition selon la revendication 15, **caractérisée en ce qu'**elle contient 1 à 5 % en poids d'huile de graines de ronce petit mûrier.

17. Composition selon l'une quelconque des revendications 1 à 3, **caractérisée en ce qu'**elle constitue une composition prévue pour l'hygiène de la peau.

18. Composition selon la revendication 17, **caractérisée en ce qu'**elle contient 0,1 à 20 % en poids d'huile de graines de ronce petit mûrier.

19. Composition selon la revendication 18, **caractérisée en ce qu'**elle contient 1 à 5 % en poids d'huile de graines de ronce petit mûrier.
